# EUROPÄISCHE PATENTSCHRIFT

(11) **EP 0 579 347 B1**
(45) Veröffentlichungstag und Bekanntmachung des Hinweises auf die Patenterteilung: **24.04.1996**
(21) Anmeldenummer: 93250193.5
(22) Anmeldetag: 29.06.1993
(51) Int. Cl.: A61K 9/16, A61K 9/50

(54) **Mikroverkapselung wasserlöslicher Wirkstoffe**
Microencapsulation of water-soluble drugs
Microencapsulation de médicaments hydrosolubles

(30) Priorität: 10.07.1992 DE 4223169
(43) Veröffentlichungstag der Anmeldung: 19.01.1994
(73) Patentinhaber: FERRING ARZNEIMITTEL GmbH, D-24109 Kiel 1 (DE)
(72) Erfinder: Nerlich, Birgit, Dr., D-2300 Kiel-Russee (DE); Mank, Reinhard, Dr., D-2300 Kiel-Russee (DE); Gustafsson, Jan, Dr., D-2300 Kiel 1 (DE); Hörig, Joachim, D-2300 Kiel-Kronshagen (DE); Köchling, Wolfgang, Dr., D-2303 Tüttendorf (DE)
(74) Vertreter: UEXKÜLL & STOLBERG

(56) Entgegenhaltungen:
- EP-A- 0 330 180
- EP-A- 0 377 477
- JOURNAL OF PHARMACEUTICAL SCIENCES Bd. 79, Nr. 10 , Oktober 1990 , WASHINGTON (US) Seiten 919 - 924 XP160400 R. WADA ET AL. 'lactic acid oligomer microspheres containing hydrophilic drugs'

## Beschreibung

Die Erfindung betrifft ein Verfahren zur Mikroverkapselung wasserlöslicher Wirkstoffe durch Phasentrennung, insbesondere die Härtung der Mikrokapseln.

Die Mikroverkapselung von pharmazeutischen Wirkstoffen mit biokompatiblen Polymeren eignet sich zur Herstellung von Präparaten mit kontrolliertem Freisetzungsverhalten. Die Mikroverkapselung von pharmazeutischen Wirkstoffen durch Phasentrennung erfolgt im allgemeinen in den folgenden Schritten:
(a) Herstellen einer Lösung des Polymeren in einem mit Wasser nicht mischbaren organischen Lösungsmittel;
(b) Zugabe einer wäßrigen Lösung des Wirkstoffes oder Dispergieren des festen Wirkstoffes in der Lösung des Polymeren;
(c) Zugabe eines sogenannten Nichtlösungsmittels (Koazervationsmittels), d.h. einer organischen Flüssigkeit, die mit dem ersten organischen Lösungsmittel mischbar und in der das verwendete Polymer nicht löslich ist. Die Zugabe des Koazervationsmittels führt zur Phasentrennung und damit zur Bildung von Embryonal- oder Rohkapseln;
(d) Härten der Rohkapseln durch Einbringen des Gemisches aus Stufe (c) in ein Härtungsmittel, das die Funktion hat, das Polymer-Lösungsmittel aus Schritt (a) aus den Mikrokapseln zu extrahieren;
(e) Abtrennen, Waschen und Trocknen der gehärteten Mikrokapseln.

Zur Härtung von Mikrokapseln sind verschiedene Lösungsmittel beschrieben worden. Beispielsweise wird in der EP 52 510-A2 sowie in der EP 172 422-A2 die Härtung von Mikrokapseln mit gesättigten flüssigen Kohlenwasserstoffen beschrieben, wie z.B. Hexan, Heptan, Cyclohexan oder Petrolether. Es hat sich jedoch gezeigt, daß sich flüssige Kohlenwasserstoffe nach der Härtung nicht vollständig aus den Mikrokapseln entfernen lassen. Der Heptangehalt gehärteter Mikrokapseln beträgt typischerweise 5 bis 15% und übertrifft damit in vielen Fällen den Wirkstoffgehalt dieser Kapseln. Zudem sind flüssige Kohlenwasserstoffe brennbar, so daß ihre Verarbeitung hohe Sicherheitsvorkehrungen erfordert.

Zur Vermeidung dieser Nachteile wurden andere Lösungsmittel als Härter verwendet. In der DE 35 36 902 A1 wird die Härtung von Mikrokapseln mit Fluor- oder Fluorhalogen-Kohlenwasserstoffen beschrieben. Diese Verbindungen lassen sich jedoch auch nur unvollständig aus den gehärteten Mikrokapseln entfernen, zudem wurde in der EP 377 477 A1 gezeigt, daß solche Lösungsmittel nicht für beliebige Polymere geeignet sind. Weiterhin sind Fluor- oder Fluorhalogen-Kohlenwasserstoffe in ökologischer Hinsicht bedenklich, was in einer drastischen Reduzierung der Anwendung dieser Stoffe durch den Gesetzgeber dokumentiert wird.

Die US 5 000 886 A beschreibt die Härtung von Mikrokapseln mit flüchtigen Silikonölen, wie z.B. Octamethylcyclotetrasiloxan. Diese Stoffe sind nicht entflammbar, besitzen nur eine geringe Toxizität und lassen sich nach der Härtung der Kapseln durch Vakuumtrocknung entfernen. Auf diese Weise läßt sich eine sehr geringe Restkonzentration des Härters in den Mikrokapseln erzielen. Allerdings sind solche Silikonöle biologisch nicht abbaubar und eignen sich daher nur bedingt zur Herstellung von Mikrokapseln für eine parenterale Applikation.

Die EP 377 477 A1 betrifft die Härtung von Mikrokapseln mit Ethyl- und Isopropylestern geradkettiger C₁₂ bis C₁₈-Fettsäuren. Diese Verbindungen zeichnen sich durch eine geringe Toxizität bei Mäusen aus und werden daher als vollständig biokompatibel angesehen. Aufgrund ihrer meist relativ hohen Erstarrungspunkte zeigen sie jedoch verarbeitungstechnische Nachteile.

Aufgabe der Erfindung ist, neue Mittel zur Härtung von Mikrokapseln bereitzustellen. Diese Mittel sollen biokompatibel und biologisch abbaubar sein, eine geringe Toxizität aufweisen und die oben genannten Nachteile bekannter Härtungsmittel vermeiden. Zudem sollen sie auf einfache Weise die Steuerung des Freisetzungsverhaltens der Mikrokapseln erlauben.

Erfindungsgemäß werden unter dem Begriff "Mikrokapsel" sowohl echte Mikrokapseln, d.h. Mikropartikel, in denen ein Wirkstoffkern von einer polymeren Matrix umgeben ist, als auch monolithische Mikrokapseln (Mikrosphären) verstanden, in denen ein Wirkstoff homogen in einer polymeren Matrix verteilt ist. Biologisch abbaubare Verbindungen werden unter den im Organismus vorherrschenden Bedingungen hydrolytisch und/oder enzymatisch vollständig zu nicht toxischen Produkten abgebaut und resorbiert.

Überraschend wurde gefunden, daß sich diese Aufgabe durch Verwendung von natürlichen und synthetischen Estern gesättigter C₄ bis C₁₈-Säuren mit mehrwertigen Alkoholen als Härtungsmittel lösen läßt. Diese Ester zeichnen sich durch hervorragende Eigenschaften bei der Härtung von Rohkapseln aus, die nach dem Prinzip der Phasentrennung erhalten wurden. Weiterhin wurde völlig unerwartet gefunden, daß sich das Freisetzungsverhalten von Mikrokapseln auch über den Gehalt des Härters in den Kapseln gezielt beeinflussen läßt. Die erfindungsgemäßen Ester mehrwertiger Alkohole zeichnen sich durch eine sehr geringe Toxizität aus und sind meist vollständig biologisch abbaubar. Diese Verbindungen sind nicht leicht entflammbar und in ökologischer Hinsicht völlig unbedenklich. Die nach dem erfindungsgemäßen Verfahren hergestellten Mikrokapseln sind frei von Alkanen, von halogenierten Kohlenwasserstoffen und von anderen biologisch nicht abbaubaren Lösungsmitteln.

Zur Mikroverkapselung pharmazeutischer Wirkstoffe wird ein biokompatibles Polymer in einem organischen Lösungsmittel (Polymer-Lösungsmittel) gelöst, das mit Wasser nicht mischbar ist. Als biokompatible Polymere kommen sowohl biologisch abbaubare als auch biologisch nicht-abbaubare Polymere in Betracht. Biologisch nicht-abbaubare Polymere eignen in erster Linie sich zur oralen Applikation, biologisch abbaubare Polymere zur parenteralen und oralen Applikation.

Besonders geeignete biologisch abbaubare Polymere sind Poly-L-lactid, Poly-D,L-lactid sowie Lactid-Glycolid-Copolymere unterschiedlicher Zusammensetzung, insbesondere Poly-D,L-lactid-co-glycolide mit einem Molverhältnis von 10:90 (PLG 10/90) bis 90:10 (PLG 90/10). Des weiteren sind Mischungen der genannten Polymere anwendbar. Als Polymer-Lösungsmittel werden beispielsweise halogenierte Kohlenwasserstoffe wie CH₂Cl₂ oder CHCl₃ verwendet.

Der zu verkapselnde pharmazeutische Wirkstoff wird entweder direkt in der Lösung des Polymeren dispergiert oder aber in einer wäßrigen Lösung zu der Polymerlösung gegeben. Das erfindungsgemäße Verfahren eignet sich zur Mikroverkapselung wasserlöslicher Stoffe, bevorzugt sind biologisch aktive Stoffe. Beispiele für solche biologisch aktiven Substanzen sind Hormone und Hormon-Freisetzungsfaktoren sowie Hormon-Antagonisten, Analgetika, Antiepileptika, Cytostatika, Antipyretika, Chemotherapeutika, Narkotika-Antagonisten und Sedativa. Besonders geeignete Wirkstoffe sind Aminosäuren, Peptide und Proteine, mit und ohne zusätzlichem Nicht-Proteinanteil. Hierzu zählen beispielsweise das Gonadotropin-Releasing-Hormon (GnRH) und seine Analoga (Agonisten und Antagonisten), Calcitonin, Wachstumshormon und Hormon der Freisetzung von Wachstumshormon, Somatostatin und seine Analoga, Parathyroid-Hormon (PTH) und seine kurzkettigen Analoga. Weiterhin geeignet sind Genprodukte sowie Gen-(DNA-) und RNA-Fragmente.

Zu der Dispersion oder Emulsion des Wirkstoffs in der Polymerlösung wird ein Koazervationsmittel gegeben, das eine Phasentrennung bewirkt und zum Ausfällen des Polymeren führt. Als Koazervationsmittel eignen sich Lösungsmittel bzw. Lösungsmittelgemische oder ein zweites nicht kompatibles Polymer, die mit dem Polymerlösungsmittel mischbar sind, selbst aber das Polymer nicht lösen. Beispiele für geeignete Koazervationsmittel sind mineralische oder pflanzliche Öle, Neutral- oder Silikonöle. Die in dieser Stufe gebildeten Roh- oder Embryonalkapseln werden im folgenden Verfahrensschritt gehärtet.

Zur Härtung bringt man die Mischung der Embryonalkapseln unter Rühren langsam in einen Überschuß des Härtungsmittels ein. Die Funktion des Härtungsmittels besteht darin, das Polymerlösungsmittel und das Koazervationsmittel aus den gebildeten Mikrokapseln zu extrahieren, ohne dabei das Polymer oder den Wirkstoff zu lösen. Anschließend werden die Mikrokapseln von dem Härtungsmittel getrennt und gewaschen.

Erfindungsgemäß werden als Härtungsmittel natürliche oder synthetische Ester gesättigter C₄ bis C₁₈-Carbonsäuren mit mehrwertigen Alkoholen (Neutralöle, fette Öle) verwendet. Bevorzugt sind Fettsäuren mit einer Kettenlänge von 8 bis 18, besonders bevorzugt von 8 bis 10 C-Atomen, wie beispielsweise Capryl- und Caprinsäure. Bevorzugte Alkohole sind die zwei- und dreiwertigen Alkohole, besonders bevorzugt sind zwei- und dreiwertige Alkohole mit 2 bis 4 C-Atomen, beispielsweise Ethylenglykol, Propylenglykol und Glycerin. Die Hydroxylgruppen der Alkohole können mit gleichen oder mit unterschiedlichen Fettsäuren verestert sein. Zu den Neutralölen zählen unter anderem die unter dem Handelsnamen Miglyol® angebotenen Ester mittelkettiger Fettsäuren mit zwei- oder dreiwertigen Alkoholen. Typische Vertreter für fette Öle sind Erdnuß-, Sesam- und Olivenöl.

Das Verhältnis von Härtungsmittel zu dem Gesamtvolumen aus Polymerlösungsmittel und Koazervationsmittel ist in weiten Bereichen variierbar. Normalerweise wird das Härtungsmittel im Überschuß eingesetzt, bevorzugt werden Verhältnisse von 10:1 bis 30:1. Die Härtungszeit beträgt etwa 30 Minuten bis 2 Stunden, aber auch andere Zeiten sind möglich.

Das erfindungsgemäße Verfahren eignet sich besonders zur Herstellung von Mikrokapseln mit einem Durchmesser von 0,1 bis 2000 µm, die Herstellung von Partikeln mit kleineren oder größeren Durchmessern ist jedoch möglich.

Die erfindungsgemäßen eingesetzten Härtungsmittel haben den Vorteil, daß sie zum einen hervorragende Härtungseigenschaften zeigen, zum anderen in physiologischer Hinsicht völlig unbedenklich sind, da es sich um klinisch getestete Öle handelt. Sie sind biologisch abbaubar und eignen sich daher auch als Suspensionsmittel für die fertigen Mikrokapseln. Die Verwendung des gleichen Mittels sowohl als Härter als auch als Suspensionsmittel erleichtert die Resuspension der Mikropartikel und macht damit die Zugabe von Hilfsstoffen, wie Tensiden, überflüssig. Eine Suspension der nach dem erfindungsgemäßen Verfahren hergestellten Mikrokapseln in wäßrigen Lösungsmitteln ist jedoch möglich. Zudem verhindern die erfindungsgemäßen Öle ein Agglomerieren der Mikrokapseln und haben so einen positiven Einfluß sowohl auf die Applikation als auch auf die Verteilung im Organismus. Weiterhin wird durch die erfindungsgemäß verwendeten Öle eine stabile Suspension während der Applikation erzielt. Besonders vorteilhaft daran ist, daß die Mikrokapseln in Form von gebrauchsfertigen Suspensionen zur Verfügung gestellt werden können. Die beschriebenen Neutralöle verhalten sich gegenüber Wirk- und Hilfsstoffen chemisch völlig indifferent, außerdem werden sie durch Luftsauerstoff nicht oxydiert, zeichen sich somit durch eine hohe Stabilität aus und sind bei tiefen Temperaturen flüssig und damit leicht zu verarbeiten. Bei fetten Ölen sollten die im Arzneibuch angegebenen Spezifikationen für eine parenterale Anwendung eingehalten werden.

Ein besonderer Vorteil des erfindungsgemäßen Verfahrens besteht darin, daß sich das Freisetzungsverhalten der in den Mikrokapseln enthaltenden Wirkstoffe durch die als Härtungsmittel verwendeten Öle gezielt steuern läßt. Bislang erfolgt eine Steuerung des Freisetzungsverhaltens über polymerspezifische Eigenschaften und die Verfahrenstechnologie.

Das erfindungsgemäße Verfahren ermöglicht zusätzlich eine Steuerung der Wirkstofffreigabe, zum einen über die Viskosität der verwendeten Öle, zum anderen über den Restgehalt der Öle in den Mikrokapseln. Ein hoher Restgehalt des zur Härtung verwendeten Öls in den Mikrokapseln verhindert ein rasches Eindringen von Wasser in die Kapseln und damit ein Herauslösen der wasserlöslichen Wirkstoffe, eine Reduzierung des Restölgehalts in den Kapseln erleichtert das Eindringen von Wasser in die Mikrokapseln und das Herauslösen der Wirkstoffe. Diese Steuerung des Freisetzungsverhaltens ist möglich, da die erfindungsgemäß als Härtungsmittel verwendeten Öle biokompatible, biologisch abbaubar und in toxikologischer Hinsicht völlig unbedenklich sind, so daß, anders als bei bekannten Härtungsmitteln, der Gehalt des Härtungsmittels in den Kapseln nicht auf ein möglichst geringes Maß reduziert werden muß. Die Freisetzung der Wirkstoffe läßt sich zeitlich steuern, beispielsweise auf einen Zeitraum von einem Monat oder länger einstellen.

Der Ölgehalt der Mikrokapseln läßt sich durch Waschen der gehärteten Mikrokapseln mit einem Lösungsmittel gezielt einstellen. Als Lösungsmittel eignen sich unter anderem Alkohole, bevorzugt ist Isopropanol.

Die erfindungsgemäßen Härtungsmittel eignen sich darüber hinaus, wie oben erwähnt, auch als Koazervationsmittel. Durch Verwendung des gleichen Stoffes als Koazervations-, Härtungs- und Suspensionsmittel läßt sich der Gehalt an unerwünschten Substanzen in den Mikrokapseln drastisch verringern.

### Beispiel 1:

Zur Mikroverkapselung wird das GnRH-Analogon Triptorelinacetat (D-Trp⁶-LHRH) als Modellwirkstoff eingesetzt. 4,4 g Poly(D,L-lactid-co-glycolid), Molverhältnis 50:50 (PLG 50/50) werden in 120 ml Methylenchlorid gelöst und in ein mit einem Rührwerk ausgerüstetes Reaktionsgefäß überführt. 0,18 g Triptorelinacetat werden in der Polymerlösung bei einer Rührgeschwindigkeit von 500 U/min suspendiert, anschließend werden unter fortgesetztem Rühren 54 g Silikonöl (Dow corning 360 Medical Fluid®) mit einer Geschwindigkeit von etwa 4,5 g/min zugegeben. Nach vollständiger Zugabe des Silikonöls wird das die Rohmikrokapseln enthaltende Gemisch in dünnem Strahl unter ständigem Rühren bei 1000 U/min kontinuierlich in 4 l eines Capryl-Caprinsäure-Triglycerids (Miglyol® 812, Viskosität 27 bis 33 mPa·s bei 20°C) überführt. Die Härtung der Mikrokapseln erfolgt in einem Zeitraum von 60 min. Die so erhaltenen Mikrokapseln werden abfiltriert, zweimal mit Isopropanol gewaschen und getrocknet.

Der Wirkstoffgehalt der Mikrokapseln wird zu 2,8% und der Ölgehalt zu 10% bestimmt. Der Teilchendurchmesser beträgt 1 bis 150 µm.

Zur Untersuchung des in-vitro-Freisetzungsverhaltens werden 90 mg der Mikrokapseln bei 37°C in 20 ml 120 mMol Phosphatpuffer (pH 7,4, 0,1% NaN₃, 0,1% Polyethylen(20)sorbitanmonolaurat (Tween® 80) suspendiert. Nach 6 h sind 4,7% des in den Mikrokapseln enthaltenden Wirkstoffes freigesetzt, nach 24 h 11,2%, nach 144 h 38,9%.

### Beispiel 2:

Analog Beispiel 1 werden Mikrokapseln hergestellt, zur Härtung wird jedoch ein Propylenglykolester der Capryl-/Caprinsäure verwendet (Miglyol® 840, Viskosität 8 bis 14 mPa·s bei 20°C).

Der Ölgehalt der Mikrokapseln beträgt vor dem Waschen mit Isopropanol 33,3%, nach einmaligem Waschen mit Isopropanol 10,7%.

Die Untersuchung des in-vitro-Freisetzungsverhaltens der gewaschenen Partikel führt zu folgenden Ergebnissen:
- 6 h:: 35,3%
- 24 h:: 57,3%
- 144 h:: 67,2%.

Im in-vivo Modell an Ratten zeigten die Mikrokapseln mit den verschiedenen Ölen eine deutliche Testosteronsuppression über 28 Tage. Dieser Effekt wurde bei Einsatz der Öle als Suspensionsmittel noch verstärkt.

### Beispiel 3:

Analog Beispiel 1 werden Mikrokapseln hergestellt, als Härtungsmittel wird Sesamöl (Arzneibuchqualität) verwendet. Das Volumen des Härtungsmittels wird auf 2 l verringert.

### Beispiel 4:

Analog Beispiel 1 werden Mikrokapseln hergestellt, als Polymer wird zum einen PLG 75/25 und zum anderen Poly-D,L-lactid eingesetzt.

### Beispiel 5:

Analog Beispiel 1 werden Mikrokapseln hergestellt, anstelle des Silikonöls wird als Koazervationsmittel ein Propylenglykolester der Capryl-/Caprinsäure (Miglyol® 840) eingesetzt.

## Patentansprüche

1. Verfahren zur Mikroverkapselung wasserlöslicher Wirkstoffe nach dem Prinzip der Phasentrennung, das die Schritte
(a) Herstellung einer Lösung eines biokompatiblen Polymeren in einem organischen Lösungsmittel;
(b) Zugabe einer wäßrigen Lösung eines oder mehrerer Wirkstoffe oder Dispersion eines oder mehrerer fester Wirkstoffe in der Lösung aus Stufe (a);
(c) Zugabe eines Koazervationsmittels zu der Emulsion oder Dispersion aus Stufe (b);
(d) Einbringen der Mischung aus Stufe (c) in einen Überschuß eines Härtungsmittels;
(e) Sammeln und Waschen der Mikrokapseln
umfaßt, **dadurch gekennzeichnet**, daß man als Härtungsmittel natürliche oder synthetische Ester gesättigter C₄ bis C₁₈-Carbonsäuren und mehrwertiger Alkohole verwendet.

2. Verfahren nach Anspruch 1, **dadurch gekennzeichnet**, daß man Ester von C₈ bis C₁₈-Fettsäuren verwendet.

3. Verfahren nach Anspruch 1, **dadurch gekennzeichnet**, daß man Ester von C₈ bis C₁₀-Fettsäuren verwendet.

4. Verfahren nach einem der Ansprüche 1 bis 4, **dadurch gekennzeichnet**, daß man Ester der Capryl- und/oder Caprinsäure verwendet.

5. Verfahren nach Anspruch 1, **dadurch gekennzeichnet**, daß man Ester zwei- oder dreiwertiger Alkohole verwendet.

6. Verfahren nach Anspruch 5, **dadurch gekennzeichnet**, daß man Ester von Ethylenglykol, Propylenglykol oder Glycerin verwendet.

7. Verfahren nach den Ansprüchen 1 bis 6, **dadurch gekennzeichnet**, daß man Ester verwendet, in denen die Hydroxylgruppen des Alkohols mit gleichen oder mit unterschiedlichen Fettsäuren verestert sind.

8. Verfahren nach Anspruch 1, **dadurch gekennzeichnet**, daß man als Ester Neutralöle oder fette Öle verwendet.

9. Verfahren nach Anspruch 8, **dadurch gekennzeichnet**, daß man als fette Öle Sesam- oder Erdnußöl verwendet.

10. Verfahren nach einem der Ansprüche 1 bis 9, **dadurch gekennzeichnet**, daß man biokompatible, biologisch nicht abbaubare oder biologisch abbaubare Polymere verwendet.

11. Verfahren nach Anspruch 10, **dadurch gekennzeichnet**, daß man als Polymer Poly-L-lactid, Poly-D,L-lactid, Lactid/glycolid-Copolymer oder Mischungen daraus verwendet.

12. Verfahren nach Anspruch 11, **dadurch gekennzeichnet**, daß man ein Lactid-/Glycolid-Copolymer mit einem Molverhältnis von Milchsäure zu Glykolsäure von 10:90 bis 90:10 verwendet.

13. Verfahren nach einem der Ansprüche 1 bis 12, **dadurch gekennzeichnet**, daß man das 10- bis 30-fache Volumen des Gesamtvolumens aus Polymerlösungsmittel und Koazervationsmittel an Härtungsmittel verwendet.

14. Verfahren nach einem der Ansprüche 1 bis 13, **dadurch gekennzeichnet**, daß man den Gehalt der Mikrokapseln an natürlichen oder synthetischen Estern gesättigter C₄- bis C₁₈-Carbonsäuren und mehrwertiger Alkohole durch Waschen mit einem Lösungsmittel gezielt verändert und dadurch die Wirkstofffreigabe steuert.

15. Verfahren nach Anspruch 14, **dadurch gekennzeichnet**, daß man als Lösungsmittel Alkohole verwendet.

16. Verfahren nach Anspruch 15, **dadurch gekennzeichnet**, daß man Isopropanol verwendet.

17. Verfahren nach einem der Ansprüche 1 bis 15, **dadurch gekennzeichnet**, daß man als Wirkstoffe pharmazeutische Wirkstoffe verwendet.

18. Verfahren nach Anspruch 17, **dadurch gekennzeichnet**, daß man als pharmazeutische Wirkstoffe Aminosäuren, Peptide, Proteine oder Hormone verwendet.

19. Verfahren nach Anspruch 18, **dadurch gekennzeichnet**, daß man als pharmazeutischen Wirkstoff Gonadotropin-Releasing-Hormon (GnRH) oder ein Analogon (Agonist oder Antagonist), Calcitonin, Wachstumshormon oder Hormon der Freisetzung von Wachstumshormon, Somatostatin oder ein Analogon, Parathyroid-Hormon (PTH) oder ein kurzkettiges Analogon verwendet.

20. Verfahren nach einem der Ansprüche 1 bis 19, **dadurch gekennzeichnet**, daß man als Koazervationsmittel den gleichen Stoff verwendet, der auch als Härtungsmittel eingesetzt wird.

21. Verwendung von Mikrokapseln, die nach einem Verfahren nach einem der Ansprüche 1 bis 20 hergestellt sind, zur Herstellung gebrauchsfertiger injizierbarer Suspensionen.

22. Verwendung nach Anspruch 21, **dadurch gekennzeichnet**, daß die gebrauchsfertige Suspension als Suspensionsmittel denselben Ester enthält, der auch zur Härtung der Mikrokapseln verwendet worden ist.

## Claims

1. Process for the microencapsulation of water-soluble active ingredients according to the principle of phase separation which comprises the steps
(a) preparation of a solution of a biocompatible polymer in an organic solvent;
(b) addition of an aqueous solution of one or more active ingredients to, or dispersion of one or more solid active ingredients in the solution from stage (a);
(c) addition of a coacervation agent to the emulsion or dispersion from stage (b);
(d) introduction of the mixture from stage (c) into an excess of a hardening agent;
(e) collection and washing of the microcapsules,
characterised by the fact that natural or synthetic esters of saturated C₄ to C₁₈ carbolyxic acids and polyhydric alcohols are used as a hardening agent.

2. Process according to claim 1, characterised by the fact that esters of C₈ to C₁₈ fatty acids are used.

3. Process according to claim 1, characterised by the fact that esters of C₈ to C₁₀ fatty acids are used.

4. Process according to one of the claims 1 to 3, characterised by the fact that esters of caprylic and/or capric acid are used.

5. Process according to claim 1, characterised by the fact that esters of di- or trihydric alcohols are used.

6. Process according to claim 5, characterised by the fact that esters of ethylene glycol, propylene glycol or glycerine are used.

7. Process according to claims 1 to 6, characterised by the fact that esters are used in which the hydroxyl groups of the alcohol are esterified with the same or with different fatty acids.

8. Process according to claim 1, characterised by the fact that neutral oils or fatty oils are used as esters.

9. Process according to claim 8, characterised by the fact that sesame oil or peanut oil are used as fatty oils.

10. Process according to one of the claims 1 to 9, characterised by the fact that biocompatible, biologically non-degradable or biologically degradable polymers are used.

11. Process according to claim 10, characterised by the fact that poly-L-lactide, poly-D,L-lactide, lactide/glycolide copolymer or mixtures thereof are used as polymer.

12. Process according to claim 11, characterised by the fact that a lactide/glycolide copolymer with a molar ratio of lactic acid to glycolic acid of between 10:90 and 90:10 is used.

13. Process according to one of the claims 1 to 12, characterised by the fact that the hardening agent is used in an amount of 10 to 30 times the total volume of polymer solvent and coacervation agent.

14. Process according to one of the claims 1 to 13, characterised by the fact that the content of natural or synthetic esters of saturated C₄ to C₁₈ carbolyxic acids and polyhydric alcohols in the microcapsules is selectively changed by washing with a solvent, thus controlling the release of the active ingredient.

15. Process according to claim 14, characterised by the fact that alcohols are used as the solvent.

16. Process according to claim 15, characterised by the fact that isopropanol is used.

17. Process according to one of the claims 1 to 15, characterised by the fact that pharmaceutical active ingredients are used as active ingredients.

18. Process according to claim 17, characterised by the fact that amino acids, peptides, proteins or hormones are used as pharmaceutical active ingredients.

19. Process according to claim 18, characterised by the fact that gonadotrophin-releasing hormone (GnRH) or an analogue (agonist or antagonist), calcitonin, growth hormone or hormone for the release of growth hormone, somastatin or an analogue, parathyroid hormone (PTH) or a short-chain analogue is used as pharmaceutical active ingredient.

20. Process according to one of the claims 1 to 19, characterised by the fact that the same substance which is used as the hardening agent is also used as the coacervation agent.

21. The use of microcapsules produced by means of a process according to one of the claims 1 to 20 for the manufacture of ready-to-use injectable suspensions.

22. Use in accordance with claim 21, characterised by the fact that the ready-to-use suspension contains, as dispersing agent, the same ester which has been used for hardening the microcapsules.

## Revendications

1. Procédé pour la microencapsulation d'agents actifs hydrosolubles selon le principe de la séparation des phases, qui comporte les étapes de :
(a) fabrication d'une solution d'un polymère biocompatible dans un solvant organique;
(b) addition d'une solution aqueuse d'un ou plusieurs agents actifs ou bien d'une dispersion d'un ou plusieurs agents actifs solides dans la solution de l'étape (a);
(c) addition d'un agent de coacervation à l'émulsion ou à la dispersion de l'étape (b);
(d) incorporation du mélange provenant de l'étape (c) dans un excès d'un agent durcisseur;
(e) récupération et lavage des microcapsules,
caractérisé en ce que l'on utilise, en tant qu'agent durcisseur, des esters naturels ou synthétiques d'acides carboniques C₄ à C₁₈ saturés et des alcools plurivalents.

2. Procédé selon la revendication 1, caractérisé en ce qu'on utilise des esters d'acides gras C₈ à C₁₈.

3. Procédé selon la revendication 1, caractérisé en ce qu'on utilise des esters d'acides gras C₈ à C₁₀.

4. Procédé selon l'une quelconque des revendications 1 à 4, caractérisé en ce qu'on utilise des esters d'acide caprylique ou caprique.

5. Procédé selon la revendication 1 caractérisé en ce qu'on utilise des esters d'alcool di- ou trivalent.

6. Procédé selon la revendication 5 caractérisé en ce qu'on utilise des esters d'éthylène glycol, de propylène glycol ou de glycérine.

7. Procédé selon les revendications 1 à 6, caractérisé en ce qu'on utilise des esters dans lesquels les groupes hydroxyles de l'alcool sont estérifiés avec des acides gras identiques ou différents.

8. Procédé selon la revendication 1 caractérisé en ce qu'on utilise, en tant qu'ester, des huiles neutres ou bien des huiles grasses.

9. Procédé selon la revendication 8, caractérisé en ce qu'on utilise en tant qu'huiles grasses de l'huile de sésame ou d'arachide.

10. Procédé selon l'une quelconque des revendications 1 à 9, caractérisé en ce qu'on utilise des polymères biocompatibles, biologiquement non dégradables ou biologiquement dégradables.

11. Procédé selon la revendication 10, caractérisé en ce qu'on utilise, en tant que polymère, le poly-L-lactide, le poly-D,L-lactide, un copolymère de lactide/glycolide ou des mélanges de ceux-ci.

12. Procédé selon la revendication 11, caractérisé en ce qu'on utilise un copolymère de lactide/glycolide ayant un rapport en moles d'acide lactique à l'acide glycolique de 10:90 à 90:10.

13. Procédé selon l'une quelconque des revendications 1 à 12, caractérisé en ce qu'on utilise 10 fois à 30 fois en volume le volume total du solvant du polymère et de l'agent de coacervation en tant qu'agent de durcisseur.

14. Procédé selon l'une quelconque des revendications 1 à 13, caractérisé en ce que l'on modifie la teneur des microcapsules en esters synthétiques ou naturels d'acides carboniques C₄ à C₁₈ saturés et alcools plurivalents par lavage avec un solvant et on contrôle ainsi la libération de l'agent actif.

15. Procédé selon la revendication 14, caractérisé en ce qu'on utilise, en tant que solvant, des alcools.

16. Procédé selon la revendication 15, caractérisé en ce qu'on utilise de l'isopropanol.

17. Procédé selon l'une quelconque des revendications 1 à 15, caractérisé en ce qu'on utilise, en tant qu'agent actif, des agents actifs pharmaceutiques.

18. Procédé selon la revendication 17, caractérisé en ce qu'on utilise, en tant qu'agent actif pharmaceutique, des acides aminés, des peptides, des protéines ou des hormones.

19. Procédé selon la revendication 18, caractérisé en ce qu'on utilise, en tant qu'agent actif pharmaceutique, l'hormone de libération de la gonadotropine (GnRH) ou bien un analogue (agoniste ou antagoniste), la calcitonine, l'hormone de croissance ou bien l'hormone de la libération de l'hormone de croissance, la somatostatine ou un analogue, l'hormone de la parathyroïde (PTH) ou un analogue à chaîne courte.

20. Procédé selon l'une des revendications 1 à 19, caractérisé en ce qu'on utilise, en tant qu'agent de coacervation, la même matière que l'agent durcisseur.

21. Utilisation de microcapsules qui sont fabriquées selon un procédé de l'une des revendications 1 à 20 pour la fabrication de suspensions injectables prêtes à l'utilisation.

22. Utilisation selon la revendication 21, caractérisée en ce que la suspension prête à l'utilisation contient, en tant qu'agent de suspension, le même ester que celui qui a été utilisé pour le durcissement des microcapsules.
